# EUROPEAN PATENT SPECIFICATION

(11) **EP 1 171 583 B1**
(45) Date of publication and mention of the grant of the patent: **21.06.2006**
(21) Application number: 00922192.0
(22) Date of filing: 12.04.2000
(51) Int. Cl.: C12N 15/10, C12N 15/52, C12N 9/10, C12N 15/63, C12N 15/90, C12P 19/62, C12R 1/465

(54) **A MULTI-PLASMID METHOD FOR PREPARING LARGE LIBRARIES OF POLYKETIDES AND NON-RIBOSOMAL PEPTIDES**
MULTI-PLASMID VERFAHREN ZUR HERSTELLUNG VON GROSSEN BIBLIOTHEKEN VON POLYKETIDEN UND NICHT-RIBOSOMALEN PEPTIDEN
METHODE MULTIPLASMIDE DE PREPARATION DE GRANDES BIBLIOTHEQUES DE POLYCETIDES ET DE PEPTIDES NON RIBOSOMIQUES

(30) Priority: 16.04.1999 US 129731 P
(43) Date of publication of application: 16.01.2002
(73) Proprietor: Kosan Biosciences, Inc., Hayward, CA 94545 (US)
(72) Inventor: SANTI, Daniel, V., San Francisco, CA 94117 (US); XUE, Qun, Charlottesville, Virginia 22903 (US); ASHLEY, Gary, Alameda, CA 94502 (US)
(74) Representative: McCall, John Douglas
(86) International application number: PCT/US2000/010021
(87) International publication number: WO 2000/063361

(56) References cited:
- WO-A-00/24907
- WO-A-00/26349
- WO-A-00/31247
- WO-A-00/52152
- WO-A-96/40968
- WO-A-97/02358
- WO-A-98/49315
- WO-A-99/05283
- C. RICHARD HUTCHINSON: "Microbial polyketide syntases: more and more prolific" PROCEEDINGS OF THE NATIONAL ACADEMY OF SCIENCES OF THE UNITED STATES, vol. 96, no. 7, 30 March 1999 (1999-03-30), pages 3336-3338, XP002151574
- MCDANIEL ROBERT ET AL: "Multiple genetic modifications of the erythromycin polyketide synthase to produce a library of novel "unnatural" natural products." PROCEEDINGS OF THE NATIONAL ACADEMY OF SCIENCES OF THE UNITED STATES, vol. 96, no. 5, 2 March 1999 (1999-03-02), pages 1846-1851, XP002143433 March 2, 1999 ISSN: 0027-8424 cited in the application
- KHOSLA C ET AL: "Generation of polyketide libraries via combinatorial biosynthesis" TRENDS IN BIOTECHNOLOGY,GB,ELSEVIER PUBLICATIONS, CAMBRIDGE, vol. 14, no. 9, 1 September 1996 (1996-09-01), pages 335-341, XP004035738 ISSN: 0167-7799
- LIU L ET AL: "Biosynthesis of 2-nor-6-deoxyerythronolide B by rationally designed domain substitutions" JOURNAL OF THE AMERICAN CHEMICAL SOCIETY,AMERICAN CHEMICAL SOCIETY, WASHINGTON, DC,US, vol. 119, no. 43, 29 October 1997 (1997-10-29), pages 10553-10554, XP002143432 ISSN: 0002-7863
- DONADIO S ET AL: "MODULAR ORGANIZATION OF GENES REQUIRED FOR COMPLEX POLYKETIDE BIOSYNTHESIS" SCIENCE,US,AMERICAN ASSOCIATION FOR THE ADVANCEMENT OF SCIENCE,, vol. 252, 3 May 1991 (1991-05-03), pages 675-679, XP002035890 ISSN: 0036-8075
- RUAN X ET AL: "Acyltransferase domain substitutions in erythromycin polyketide synthase yield novel erythromycin derivatives" JOURNAL OF BACTERIOLOGY,US,WASHINGTON, DC, vol. 179, no. 20, October 1997 (1997-10), pages 6416-6425, XP002117397 ISSN: 0021-9193
- KAO C M ET AL: "ENGINEERED BIOSYNTHESIS OF A COMPLETE MACROLACTONE IN A HETEROLOGOUS HOST" SCIENCE,US,AMERICAN ASSOCIATION FOR THE ADVANCEMENT OF SCIENCE,, vol. 265, no. 5171, 22 July 1994 (1994-07-22), pages 509-512, XP002073974 ISSN: 0036-8075
- XUE YONGQUAN ET AL: "A gene cluster for macrolide antibiotic biosynthesis in Streptomyces venezuelae: Architecture of metabolic diversity" PROCEEDINGS OF THE NATIONAL ACADEMY OF SCIENCES OF USA,US,NATIONAL ACADEMY OF SCIENCE. WASHINGTON, vol. 95, no. 21, 13 October 1998 (1998-10-13), pages 12111-12116, XP002117399 ISSN: 0027-8424
- GRAZIANI E I ET AL: "Macrolide biosynthesis: a single cytochrome P450, PicK, is responsible for the hydroxylations that generate methymycin, neomethymycin, and picromycin in streptomyces venezuelae" BIOORGANIC & MEDICINAL CHEMISTRY LETTERS,GB,OXFORD, vol. 8, no. 22, 17 November 1998 (1998-11-17), pages 3117-3120, XP004143711 ISSN: 0960-894X
- BETLACH M C ET AL: "Characterization of the macrolide P-450 hydroxylase from Streptomyces venezuelae which converts narbomycin to picromycin" BIOCHEMISTRY,US,AMERICAN CHEMICAL SOCIETY. EASTON, PA, vol. 37, no. 42, 20 October 1998 (1998-10-20), pages 14937-14942, XP002117398 ISSN: 0006-2960
- OLANO C ET AL: "Analysis of a Streptomyces antibioticus chromosomal region involved in oleandomycin biosynthesis, which encodes two glycosyltransferases responsible for glycosylation of the macrolactone ring" MOLECULAR AND GENERAL GENETICS,DE,SPRINGER VERLAG, BERLIN, vol. 259, no. 3, 1 August 1998 (1998-08-01), pages 299-308, XP002096258 ISSN: 0026-8925
- QUIROS ET AL: "Biosynthesis of the macrolide oleandomycin by Streptomyces antibioticus" JOURNAL OF BIOLOGICAL CHEMISTRY,US,AMERICAN SOCIETY OF BIOLOGICAL CHEMISTS, BALTIMORE, MD, vol. 270, no. 31, 4 August 1995 (1995-08-04), pages 18234-18239, XP002096256 ISSN: 0021-9258
- SCHNEIDER A ET AL: "Targeted alteration of the substrate specificity of peptide synthetases by rational module swapping" MOLECULAR AND GENERAL GENETICS,DE,SPRINGER VERLAG, BERLIN, vol. 257, no. 3, February 1998 (1998-02), pages 308-318, XP002113933 ISSN: 0026-8925
- MARAHIEL M A ET AL: "Modular ppetide synthetases involved in nonribosomal peptide synthesis" CHEMICAL REVIEWS,US,AMERICAN CHEMICAL SOCIETY. EASTON, vol. 97, no. 7, November 1997 (1997-11), pages 2651-2673, XP002133489 ISSN: 0009-2665
- TANG LI ET AL: "Cloning and heterologous expression of the epothilone gene cluster" SCIENCE,US,AMERICAN ASSOCIATION FOR THE ADVANCEMENT OF SCIENCE, vol. 287, 28 January 2000 (2000-01-28), pages 640-642, XP002135841 ISSN: 0036-8075
- TANG L ET AL: "FORMATION OF FUNCTIONAL HETEROLOGOUS COMPLEXES USING SUBUNITS FROM THE PICROMYCIN, ERYTHROMYCIN AND OLEANDOMYCIN POLYKETIDE SYNTHASES" CHEMISTRY AND BIOLOGY,GB,CURRENT BIOLOGY, LONDON, vol. 7, no. 2, February 2000 (2000-02), pages 77-84, XP000909347 ISSN: 1074-5521
- XUE QUN ET AL: "A multiplasmid approach to preparing large libraries of polyketides." PROCEEDINGS OF THE NATIONAL ACADEMY OF SCIENCES OF THE UNITED STATES, vol. 96, no. 21, 12 October 1999 (1999-10-12), pages 11740-11745, XP002151575 Oct. 12, 1999 ISSN: 0027-8424

## Description

### Field of the Invention

The present invention provides recombinant DNA compounds and host cells containing novel polyketide synthase (PKS) genes and novel polyketides. The invention relates to the fields of chemistry, medicinal chemistry, human and veterinary medicine, molecular biology, pharmacology, agriculture, and animal husbandry.

### Background of the Invention

Polyketides are structurally diverse natural products that include important therapeutic agents used as antibacterials (erythromycin), immunosuppressants (FK506), cholesterol-lowering agents (lovastatin), and others (see Katz *et al.,* 1993, Polyketide synthesis: prospects for hybrid antibiotics, *Annu. Rev. Microbiol.* **47:** 875-912, ). Currently, there are about 7,000 identified polyketides, but this represents only a small fraction of what nature is capable of producing.

DNA sequencing of genes encoding several of the enzymes that produce type 1 modular polyketide synthases (PKSs) has revealed the remarkably logical organization of these multifunctional enzymes (see Cortes *et al.,* 1990, An unusually large multifunctional polypeptide in the erythromycin-producing polyketide synthase of *Saccharopolyspora erythraea, Nature* **348**:176-178; Donadio *et al.,1991,* Modular organization of genes required for complex polyketide biosynthesis, *Science* 252: 675-679; Schwecke *et al.,1995,* The biosynthetic gene cluster for the polyketide immunosuppressant rapamycin, *Proc. Natl. Acad. Sci. USA* 92: 7839-7843; and August *et al.,1998,* Biosynthesis of the ansamycin antibiotic rifamycin: deductions from the molecular analysis of the rif biosynthetic gene cluster of *Amycolatopsis mediterranei* S699, *Chem Biol* 5: 69-79,

The application of innovative combinatorial techniques to this genetic organization has prompted the generation of novel natural products, by adding, deleting, or exchanging domains or entire modules. See U.S. Patent Nos. 5,672,491; 5,712,146; 5,830,750; 5,843,718; 5,962,290; and 6,022,731, It would be advantageous to have a practical combinatorial biosynthesis technology that could achieve and perhaps exceed the diversity of modular polyketide structures thus far revealed in nature.

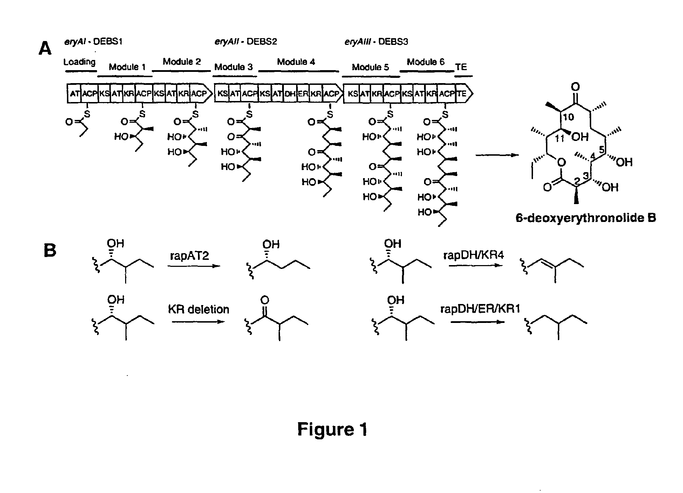

The known modular PKSs have a linear organization of modules, each of which contains the activities needed for one cycle of polyketide chain elongation, as illustrated for 6-deoxyerythronolide B synthase (DEBS) in Fig. 1A. The minimal module contains a ketosynthase (KS), an acyltransferase (AT), and an acyl carrier protein (ACP) that together catalyze a 2-carbon extension of the chain. The specificity of the AT for either malonyl or an alpha-alkyl malonyl CoA determines which 2-carbon extender is used, and thus the nature of the alkyl substituent at the alpha-carbon of the growing polyketide chain. After each 2-carbon unit condensation, the oxidation state of the beta-carbon is either retained as a ketone, or modified to a hydroxyl, methenyl, or methylene group by the presence a ketoreductase (KR), a KR + a dehydratase (DH), or a KR + DH + an enoyl reductase (ER), respectively. In effect, the AT specificity and the composition of catalytic domains within a module serve as a "code" for the structure of each 2-carbon unit. The order of the modules in a PKS specifies the sequence of the distinct 2-carbon units, and the number of modules determines the size of the polyketide chain.

The remarkable structural diversity of polyketides (see O'Hagan, *The Polyketide Metabolites;* Ellis Horwood, Chichester, 1991, is governed by the combinatorial possibilities of arranging modules containing the various catalytic domains, the sequence and number of modules, and the post-PKS "tailoring enzymes" that accompany the PKS genes. The direct correspondence between the catalytic domains of modules in a PKS and the structure of the resulting biosynthetic product allows rational modification of polyketide structure by genetic engineering.

Over the past several years, examples of modifying each of the elements that code for polyketide structure has been accomplished (see Kao *et al.,* 1996, Evidence for two catalytically independent clusters of active sites in a functional modular polyketide synthase, *Biochemistry* 35: 12363-12368; Liu *et al.,1997,* Biosynthesis of 2-nor-6-deoxyerythronolide B by rationally designed domain substitution, J. *Am. Chem. Soc.* 119: 10553-10554; McDaniel *et al.,* 1997, Gain-of-function mutagenesis of a modular polyketide synthase, J. *Am. Chem. Soc. 119:* 4309-4310; Marsden *et al.,* 1998, Engineering broader specificity into an antibiotic-producing polyketide synthase, *Science* 279: 199-202; and Jacobsen *et al.,* 1997, Precursor-directed biosynthesis of erythromycin analogs by an engineered polyketide synthase, *Science* 277: 367-369,

Recently, a combinatorial library of over 50 novel polyketides was prepared by systematic modification of DEBS, the PKS that produces the macrolide aglycone precursor of erythromycin (see U.S. patent application Serial No. 09/429,349, filed 28 Oct. 1999; PCT patent application US99/24483, filed 20 Oct. 1999; and McDaniel *et al.,* 1999, Multiple genetic modification of the erythromycin gene cluster to produce a library of novel "unnatural" natural products, *Proc. Natl. Acad. Sci. USA* 96: 1846-1851. With a single plasmid containing the *eryAI, -AII* and *-AIII* genes encoding the three DEBS subunits, ATs and beta-carbon processing domains were substituted by counterparts from the rapamycin PKS (see Schwecke *et al.,1995, supra)* that encode alternative substrate specificities and beta-carbon processing activities. The approach used was to develop single "mutations", then sequentially combine the single mutations to produce multiple changes in the PKS. It was observed that when two or more single PKS mutants were functional, there was a high likelihood that combinations would also produce the expected polyketide. Although this strategy provided high assurance that the multiple mutants would be productive, the production of each polyketide required a separate engineering. Thus, if X mutants of *eryAI,* Y mutants of *eryAII,* and Z mutants at *eryAIII* were prepared, X+Y+Z separate experiments were required to produce that same number of polyketides. Clearly, the preparation of very large libraries by this approach is laborious.

Another strategy for preparing large numbers of polyketides is by random digestion-religation leading to "mutagenesis" of the domains or modules of a mixture of PKS genes, including the refinements embodied in the DNA shuffling method (see Patten *et al.,* 1997, Applications of DNA shuffling to pharmaceuticals and vaccines, *Curr. Op. Biotechnol.* 8: 724-733,). The expected low probability of assembling an active PKS by such an approach, however, would demand an extraordinary analytical effort (in the absence of a biological selection) to detect clones that produced polyketides within the much larger number of clones that are non-producers.

There remains a need for practical approaches to create large libraries of polyketides, non-ribosomal peptides, and mixed polyketides/non-ribosomal peptides.

### Summary of the Invention

The present invention provides a method for expressing a polyketide or non-ribosomal peptide in a host cell employing a multiplicity of recombinant vectors, which may be integrative or freely replicating. Each of the multiplicity of vectors encodes a portion of the polyketide synthase or non-ribosomal peptide synthase that produces the polyketide or non-ribosomal peptide. In one embodiment, at least one of the multiplicity of vectors encodes one or more proteins that further modify the polyketide or non-ribosomal peptide produced.

In a preferred embodiment, the vectors replicate in and/or integrate into the chromosome of a *Streptomyces* host cell. Preferred integrating vectors include vectors derived from pSET152 and pSAM2. Preferred replicating vectors include those containing a replicon derived from SCP2* or pJV1.

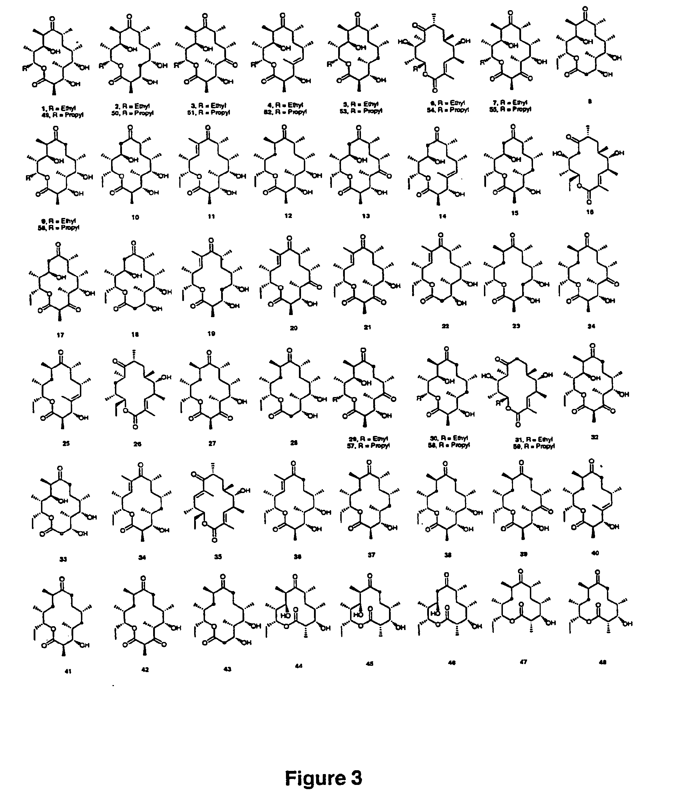

In another embodiment, the present invention provides novel polyketides. Such novel polyketides include those shown in Figure 3 as compound nos. 29-33, 35-42 and 45-59. Other novel polyketides of the invention include the polyketides obtainable by hydroxylation and/ or glycosylation of compounds 29-33, 35-42 and 45-59. Preferred compounds of the invention include those 14-membered macrolactones with a C-6 and/ or C-12 hydroxyl and/ or a C-3 and/ or C-5 glycosyl, including but not limited to those with a desosaminyl residue at C-5 and a cladinosyl residue at C-3.

These and other embodiments, modes, and aspects of the invention are described in more detail in the following description, the examples, and claims set forth below.

### Description of the Drawings

Figure 1 shows wild-type and mutant forms of the *eryA* genes and DEBS proteins. Part A depicts the *eryAI-eryAIII* genes and proteins as broad arrows oriented in the direction of transcription with the domains in modules 1 to 6 of DEBS1-DEBS3 indicated by the symbols defined herein. The first substrate, propionyl-CoA, is attached to the loading domain ACP and (2S)-2-methylmalonyl-CoA to the module 2 ACP. Then, a decarboxylative condensation between the propionate and methylmalonate takes place followed by reduction of the incipient beta-ketone to form the intermediate shown attached to the ACP of module 2. This intermediate is transferred to the ACP of module 3, and the sequence of reactions is repeated at each of the other modules with or without ketone reduction, dehydration, or double bond reduction to form the linear 21-carbon polyketide attached to the ACP of module 6. The linear polyketide then is cyclized and released as 6-deoxyerythronolide B (6dEB). Part B depicts replacement of the one or more of the domains in DEBS1, DEBS2, or DEBS3 with one of the three rap (rapamycin) PKS domains or cassettes, or deletion of the KR. This results in the corresponding functional group changes shown at one or more of the positions of 6dEB.

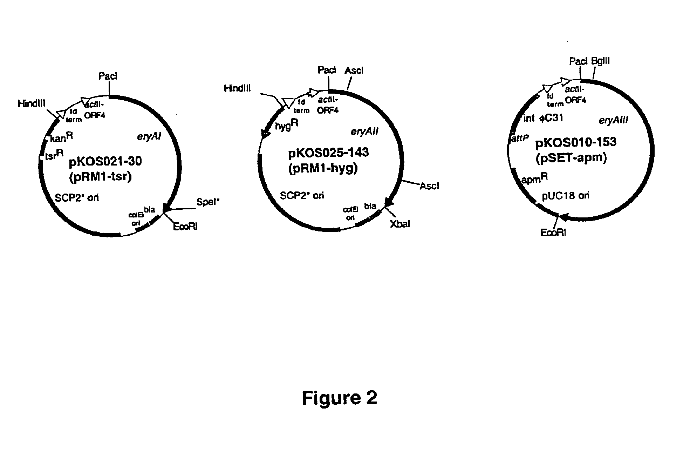

Figure 2 shows an illustrative three-plasmid expression system for *eryA* genes. In each vector, the *eryA* gene is expressed under the control of the upstream *actI* promoter and *actII-ORF4* gene as previously described (see Ziermann *et al.,* 2000, A two-vector system for the production of recombinant polyketides in *Streptomyces, J. Ind. Microbiol.* & *Biotech.* **24**: 46-50; and Kao *et al.,* 1994, Engineered biosynthesis of a complete macrolactone in a heterologous host, *Science* 265: 509-512.To facilitate construction of the various *eryA* mutations, a *Spe*I site (ACTAGT) was introduced at nt 10366-10371 of the *eryAI* ORF by making D3455T and A3456S mutations (see Kao *et al.,* 1995, Manipulation of macrolide ring size by directed mutagenesis of a modular polyketide synthase, J. *Am. Chem. Soc.* 117: 9105-9106, in pKOS025-179. This change enables insertion of the mutated gene segment between the *PacI* site and the *Spe*I site of pKOS025-179. After the replacement of the 6-kb fragment between the *Asc*I sites in *eryAII* (nt 1213 and nt 7290) with a 6-kb *Asc*I fragment containing a specific AT substitution in an intermediate plasmid, the resulting *Pac*I*-Xba*I fragment containing the mutant *eryAII* gene was inserted into pKOS025-143. All eryAIII mutants were constructed by replacing the segment in pKOS010-153 between the unique *Bgl*II site at nt 251 and the EcoRI site (nt 9290) that overlaps the stop codon.
Figure 3 shows structures of macrolactones produced by *Streptomyces lividans* strains containing assorted combinations of three plasmids (Fig. 2 and Table 1). The positions in 6dEB (1) that are altered correspond to the genetic characteristics of modules 2, 3, 5 and 6 of DEBS, as illustrated in Fig.1A and Fig. 2, and listed in Table 1. Structures 1- 40 and 49 - 56 are 14-membered lactones, and structures 44 to 48 are 12-membered lactones. Compounds 49 - 59 with the C13 propyl group were produced by mutational biosynthesis with the *eryAI* KS1° null allele.

### Detailed Description of the Invention

The present invention provides methods and reagents for using multiple recombinant DNA vectors to produce polyketides in recombinant host cells. In an illustrative embodiment, a three-plasmid system for heterologous expression of 6-deoxyerythronolide B synthase (DEBS) is provided to facilitate combinatorial biosynthesis of polyketides made by type I modular polyketide synthases (PKSs). The *eryA* PKS genes encoding the three DEBS subunits were individually cloned into three compatible *Streptomyces* vectors carrying mutually selectable antibiotic resistance markers. A strain of *Streptomyces lividans* transformed with all three plasmids produced 6-deoxyerythronolide B at a level similar to that of a strain transformed with a single plasmid containing all three genes.

The utility of this system in combinatorial biosynthesis was demonstrated through production of a library of modified polyketide macrolactones, using versions of each plasmid constructed to contain defined mutations. Combinations of these vector sets were introduced into *Streptomyces lividans,* resulting in strains producing a wide range of 6-deoxyerythronolide B analogs. This method can be applied to any modular PKS or non-ribosomal peptide synthase (NRPS) and has the potential to produce thousands of novel natural products, including ones derived from further modification of the PKS or NRPS products by tailoring enzymes.

Thus, in one aspect, the present invention provides a method for using multiple (two, three, four, or more) recombinant DNA vectors, each encoding a portion of a PKS, NRPS, or tailoring enzyme, to produce a polyketide or non-ribosomal peptide or a mixed polyketide/non-ribosomal peptide in a host cell. In one embodiment, the vectors in combination encode a naturally occurring PKS or NRPS and the corresponding natural product is produced. In another embodiment, the vectors in combination encode naturally occurring proteins from two or more different naturally occurring PKS or NRPS. In another embodiment, at least one of the vectors encodes a protein not found in nature, having been altered by recombinant DNA methodology to change its structure and function.

In one embodiment, the present invention provides a method for creating a polyketide library that enables the production of large libraries of polyketides while retaining the high probability of obtaining productive clones by combining PKS mutations known to be productive. The principle involves cloning mutants of individual open reading frames (ORFs) of a PKS on separate compatible plasmids, then coexpressing the separate ORFs in a suitable host to produce the PKS. Using this multiple plasmid approach, with X mutants of ORF 1, Y mutants of ORF 2, and Z mutants of ORF 3, for instance, a combinatorial library of X x Y x Z mutants can be achieved expeditiously.

DEBS was chosen to illustrate this multiple plasmid approach. The DEBS PKS consists of three >280 kD protein subunits, each containing two modules, that are assembled into the complete PKS complex (see Donadio *et al., 1991, supra).* With the two possible AT domains and the four possible beta-keto modifications, i.e. eight at each module, therefore 8 x 8 = 64 permutations for the two modules are possible in each DEBS subunit. Constructing the mutations in each DEBS ORF separately would require that 64 manipulations be carried out on each gene, or a total of 192 such manipulations. However, by co-transforming a host strain with three plasmids, each bearing the 64 permutations of a different DEBS subunit, one could generate the mutant PKSs necessary to achieve, in theory, a library of 262,144 polyketides (64³), as 6-deoxyerythronolide B (6dEB) analogs (Fig.1A). In contrast, the same number of mutagenesis experiments performed in a single plasmid system would theoretically yield only 192 polyketides.

The successful implementation of this multiple plasmid strategy requires that the DEBS subunits translated from three different mRNAs faithfully interact to give the active PKS. Some indication that this would be the case was provided by in vitro experiments that showed that reconstitution of the isolated DEBS1-DEBS2 complex with DEBS3 forms a functional PKS (see Pieper *et al.,* 1995, Cell-free synthesis of polyketides by recombinant erythromycin polyketide synthases, *Nature* **378**: 263-266. Further, it was recently demonstrated that coexpression of the three subunits of DEBS from two plasmids produced active PKS *in vivo* (see Ziermann *et al.,* 2000, *supra).* The present invention can be applied to any PKS or NRPS, including but not limited to the PKS enzymes, including the KS1 null mutation containing versions, that synthesize oleandolide and megalomicin (see U.S. patent application Serial Nos. 60/158,305, filed 8 Oct. 1999 and 09/428,517, filed 28 Oct. 1999, and PCT application No. US99/24478, filed 22 Oct. 1999,

The method in one embodiment requires at least three vectors that can be separately introduced into a *Streptomyces* or other suitable host strain and concomitantly express functional PKS subunits. Two such vectors are preferred for such purposes: the autonomously replicating SCP2*-based plasmid pRM1 (see Kao *et al.,* 1994, Engineered biosynthesis of a complete macrolactone in a heterologous host, *Science* 265: 509-512, and U.S. Patent No. 6,022,731, and the integrating bacteriophage phiC31-based plasmid pSET152 (see Bierman *et al.,* 1992, Plasmid cloning vectors for the conjugal transfer of DNA from *Escherichia coli* to *Streptomyces* spp., *Gene* 116: 43-49, Several additional plasmids have been tested as described herein and can be used in accordance with the present invention.

Each of these additional plasmids was tested using constructs that contained identical configurations of an *eryA* gene downstream of the *Streptomyces coelicolor actI* promoter and *actII-*ORF4 transcriptional activator as described (see Ziermann *et al.,* 2000, *supra).* Plasmid pB45, a high copy replicating plasmid possessing the pJV1 origin (see Servin-Gonzalez *et al.,* 1995, Sequence and functional analysis of the *Streptomyces phaechromogenes* plasmid pJV1 reveals a modular organization of *Streptomyces* plasmids that replicate by rolling circle, *Microbiology* 141: 2499-2510, carrying *eryAII* was introduced into *S. lividans* harboring *eryAI* on pRM1 and *eryAIII* in the pSET152 integration site; less than 0.1 mg/L of 6dEB was produced in this system compared with 50 mg/L for the single plasmid system (see Kao *et al.,* 1994, supra).

The multiple vectors used in the present method can include two or more different vectors that share the same origin of replication. For example, two SCP2*-type plasmids carrying different antibiotic markers can coexist and express PKS subunits in a *Streptomyces* sp., as has been reported using high-copy number plasmids (see Rajgarhia *et al.,* 1997, Minimal *Streptomyces* sp. strain C5 daunorubicin polyketide biosynthesis genes required for aklanonic acid biosynthesis, J. *Bacteriol.* 179: 2690-2696,. Co-transformation of S. *lividans* with *eryAIII*/pSET*-apm, eryAI*/pRM1*-tsr* and *eryAII*/pRM1-*hyg* (Fig. 2) yielded a strain that also produced 50 mg/L of 6dEB. Further, after over 24 generations under double antibiotic selection, both replicating plasmids could be rescued by standard procedures with unchanged restriction maps (see Hopwood *et al.,* 1985, *Genetic Manipulation of Streptomyces. A laboratory manual.* John Innes Foundation, Norwich,

The multiple vectors of the present invention can include two or more different integrating vectors as well. For example, the *eryAII* gene was cloned into a pSAM2 site-specific integrating plasmid (see Smokvina *et al.,1990,* Construction of a series of pSAM2-based integrative vectors for use in *Actinomycetes, Gene* 94: 53-59, Sequential transformation of *Streptomyces lividans with eryAIII*/*pSET-apm, eryAII*/*pSAM-hyg* and *eryAI*/pRM1-*tsr* provided a strain that produced 40-50 mg/L of 6dEB. A potential advantage of this system over the two-replicating vector system is that it requires one fewer antibiotic, and avoids potential problems in maintaining two plasmids containing the same *ori* in a *Streptomyces* host (see Baltz,1997, Molecular genetic approaches to yield improvement in *Actinomycetes, Biotechnology of Antibiotics,* 2nd. Edition: pp. 49-62, ). However, as shown herein, the two SCP2*-based plasmids and the pSET-derived vector can also be used.

Thus, the invention can be practiced with a wide variety of expression vectors for use in *Streptomyces.* The replicating expression vectors of the present invention include, for example and without limitation, those that comprise an origin of replication from a low copy number vector, such as SCP2* (see Hopwood *et al., Genetic Manipulation of Streptomyces: A Laboratory manual* (The John Innes Foundation, Norwich, U.K., 1985); Lydiate *et al.,1985, Gene 35: 223-235;* and Kieser and Melton, 1988, *Gene* 65: 83-91, each of which is SLP1.2 (Thompson *et al.,* 1982, *Gene 20:* 51-62, and pSG5(ts) (Muth *et al.,1989, Mol. Gen. Genet.* 219: 341-348, and Bierman *et al.,1992, Gene* 116: 43-49, or a high copy number vector, such as pIJ101 and pJV1 (see Katz *et al.,* 1983, J. *Gen. Microbiol.* 129: 2703-2714; Vara *et al.,* 1989, J. *Bacteriol.* 17I: 5782-5781; and Servin-Gonzalez, 1993, *Plasmid* 30:131-140, High copy number vectors are, however, generally not preferred for expression of large genes or multiple genes. For non-replicating and integrating vectors and generally for any vector, it is useful to include at least an *E*. *coli* origin of replication, such as from pUC, p1P, p1I and pBR. For phage based vectors, the phage phiC31 and its derivative KC515 can be employed (see Hopwood *et al., supra).* Also, plasmid pSET152, plasmid pSAM, plasmids pSE101 and pSE211, all of which integrate site-specifically in the chromosomal DNA of S. *lividans,* can be employed for purposes of the present invention.

Moreover, a wide variety of selectable markers can be employed in the *Streptomyces* recombinant expression vectors of the invention. These include antibiotic resistance conferring genes selected from the group consisting of the *ermE* (confers resistance to erythromycin and lincomycin), *tsr* (confers resistance to thiostrepton), *aad*A (confers resistance to spectinomycin and streptomycin), *aacC4* (confers resistance to apramycin, kanamycin, gentamicin, geneticin (G418), and neomycin), hyg (confers resistance to hygromycin), and *vph* (confers resistance to viomycin) resistance conferring genes. Alternatively, several polyketides are naturally colored, and this characteristic can provide a built-in marker for identifying cells.

An illustrative library of the present invention was constructed. The library was composed of vectors encoding three single mutations in *eryAI* (module 2), one in *eryAII* (module 3), and seven in *eryAIII* (modules 5 or 6) as well as wild-type ORFs, a KS1 null mutant, and a module 6 deletion, as shown in Table 1, below. To facilitate cloning, vectors were prepared that contained restriction sites that allowed transfer of DNA cassettes from previously prepared mutant *eryA* genes (see McDaniel *et al.,* 1999, *supra,* and Fig. 2). Fourteen of these expression vectors, comprising the three wild-type and eleven mutant ORFs were constructed by cassette transfers from plasmids previously prepared in the single plasmid system.

**Table 1 Genotype of the plasmids containing DEBS genes^{a}**

| Vector: | pKOS021 | pKOS025 | | pKOS010 |
|---|---|---|---|---|
| Gene: *eryAI*(DEBS1) | | *eryAII*(DEBS2) | *eryAIII*(DEBS3) | |
| Module: 1 | | 3 | 5 | 6 |
| **1.** | **wild-type** | | | |
| 2. | AT->^{b}rapAT2 | | | |
| 3. | KR->rapDH/KR4 | | | |
| 4. | KR->rapDH/ER/KR1 | | | |
| 5. | KS1^{°c} | | | |
| | | **6. wild-type** | | |
| | | 7. AT3->rapAT2 | | |
| | | | **8.** | **wild-type** |
| | | | 9. | AT->rapAT2 |
| | | | 10. | KR->AT/ACP linker |
| | | | 11. | KR->rapDH/KR4 |
| | | | 12. | KR->rapDH/ER/KR1 |
| | | | 13. | module 5 + TE^{d} |
| | | | | 14. AT->rapAT2 |
| | | | | 15. KR->AT/ACP linker |
| | | | | 16. KR->rapDH/KR4 |

| | | | | |
|---|---|---|---|---|
| a The plasmids with mutant DEBS genes were constructed by cloning the specified DNA segments into one of the three vectors shown in Fig. 2. | | | | |
| ^{b} The arrow signifies that the wild-type domain was replaced with the one indicated. | | | | |
| c The Cys729 Ala null allele was created by site-specific mutagenesis. | | | | |
| ^{d} Module 6 was deleted to fuse its TE to the ACP of module 5. | | | | |

In *eryAI,* module 2 was modified by replacing the AT by rapAT2 (the AT domain of module 2 of the rapamycin PKS), and the KR by rapDH/KR4 (the DH and KR domains of module 4 of the rapamycin PKS) or rapDH/ER/KR1 (the DH, KR, and ER domains of module 1 of the rapamycin PKS). In *eryAII,* the AT of module 3 was replaced by rapAT2; in *eryAIII,* module 5 was modified by replacing the AT by rapAT2, and the KR by rapDH/KR4 or rapDH/ER/KR1 or the AT/ ACP linker that eliminates the ery module 5 KR activity. Also, in module 6 the AT was replaced with rapAT2, and the KR by rapDH/KR4 or the AT/ ACP linker. The consequence of introducing each of these rapamycin PKS gene cassettes into DEBS is shown in Fig.1B; at a given alpha-position in the growing polyketide carbon chain, a methyl group can be removed, or a beta-hydroxyl can be changed to a ketone or removed by dehydration to produce a double bond, or the double bond resulting from dehydration can then be reduced to a methylene group.

The approach employed was to first introduce the eight *eryAIII* variants individually into the pSET integration site of *S. lividans,* then to cotransform the resulting strains individually with each of four *eryAI* variants on SCP2*-tsr vectors and two *eryAII* variants on SCP2*-hyg vectors. Thus, from the 14 vectors prepared, 64 triple transformants were obtained. Recombinant cells were grown under appropriate antibiotic selection, and extracts analyzed for polyketide production by LC/MS. Of the 64 triple transformants, 46 (72%) produced detectable levels of one or more polyketides under the test conditions employed (Fig. 3), and 43 different polyketides were produced. These included 6dEB (1), and products arising from 11 single (2-12), 26 double (13-38) and five (39-43) triple mutants of DEBS. Twenty-eight (1-28) of the 43 polyketides produced have been previously prepared using the single-plasmid system. Fifteen (29-43) were novel polyketides readily identifiable by mass spectra and correspondence to the products expected based on the cassettes used in the mutagenesis.

In one aspect, the present invention provides these novel polyketides. These novel polyketides can be further modified by tailoring enzymes, including the tailoring enzymes of *Saccharopolyspora erythraea.* There are a wide variety of diverse organisms that can modify macrolide aglycones to provide compounds with, or that can be readily modified to have, useful activities. For example, *Saccharopolyspora erythraea* can convert 6-dEB or derivatives thereof to a variety of useful compounds. The erythronolide 6-dEB is converted by the *eryf* gene product to erythronolide B, which is, in turn, glycosylated by the *eryB* gene product to obtain 3-O-mycarosylerythronolide B, which contains L-mycarose at C-3. The enzyme *eryC* gene product then converts this compound to erythromycin D by glycosylation with D-desosamine at C-5. Erythromycin D, therefore, differs from 6-dEB through glycosylation and by the addition of a hydroxyl group at C-6. Erythromycin D can be converted to erythromycin B in a reaction catalyzed by the *eryG* gene product by methylating the L-mycarose residue at C-3. Erythromycin D is converted to erythromycin C by the addition of a hydroxyl group at C-12 in a reaction catalyzed by the *eryK* gene product. Erythromycin A is obtained from erythromycin C by methylation of the mycarose residue in a reaction catalyzed by the *eryG* gene product. The aglycone compounds provided by the present invention, such as, for example, the compounds produced in *Streptomyces lividans,* can be provided to cultures of S. *erythraea* and converted to the corresponding derivatives of erythromycins A, B, C, and D. To ensure that only the desired compound is produced, one can use an S. *erythraea eryA* mutant that is unable to produce 6-dEB but can still carry out the desired conversions (Weber *et al.,* 1985, J. *Bacteriol. 164*(1): 425-433). Also, one can employ other mutant strains, such as *eryB, eryC, eryG,* and/ or *eryK* mutants, or mutant strains having mutations in multiple genes, to accumulate a preferred compound. The conversion can also be carried out in large fermentors for commercial production.

Moreover, there are other useful organisms that can be employed to hydroxylate and/ or glycosylate the compounds of the invention. As described above, the organisms can be mutants unable to produce the polyketide normally produced in that organism, the fermentation can be carried out on plates or in large fermentors, and the compounds produced can be chemically altered after fermentation. Thus, *Streptomyces venezuelae,* which produces picromycin, contains enzymes that can transfer a desosaminyl group to the C-5 hydroxyl and a hydroxyl group to the C-12 position. In addition, S. *venezuelae* contains a glucosylation activity that glucosylates the 2'-hydroxyl group of the desosamine sugar. This latter modification reduces antibiotic activity, but the glucosyl residue is removed by enzymatic action prior to release of the polyketide from the cell. Another organism, S. *narbonensis,* contains the same modification enzymes as S. *venezuelae,* except the C-12 hydroxylase. Thus, the present invention provides the compounds produced by hydroxylation and glycosylation of the macrolide aglycones of the invention by action of the enzymes endogenous to S. *narbonensis* and S. *venezuelae.*

Other organisms suitable for making compounds of the invention include *Micromonospora megalomicea,* which produces megalomicin A. Megalomicin A contains the complete erythromycin C structure, and its biosynthesis also involves the additional formation of megosamine (L-rhodosamine) and its attachment to the C-6 hydroxyl, followed by acylation of the C-3"' and(or) C-4"' hydroxyls as the terminal steps. Other organisms useful in converting the aglycones of the present invention to modified compounds include *Streptomyces antibioticus, S. fradiae,* and *S. thermotolerans. S. antibioticus* produces oleandomycin and contains enzymes that hydroxylate the C-6 and C-12 positions, glycosylate the C-3 hydroxyl with oleandrose and the C-5 hydroxyl with desosamine, and form an epoxide at C-8-C-8a. *S. fradiae* contains enzymes that glycosylate the C-5 hydroxyl with mycaminose and then the 4'-hydroxyl of mycaminose with mycarose, forming a disaccharide. S. *thermotolerans* contains the same activities as S. *fradiae,* as well as acylation activities. Thus, the present invention provides the compounds produced by hydroxylation and glycosylation of the macrolide aglycones of the invention by action of the enzymes endogenous to S. *antibioticus, S. fradiae,* and S. *thermotolerans.* Moreover, these and other tailoring enzymes can be cloned and incorporated into one or more of the multiple vectors used in accordance with the methods of the present invention to create libraries of modified polyketide compounds.

The resulting compounds can be further modified by synthetic chemistry, i.e., to yield the corresponding ketolides, useful as antibiotics (see, e.g., U.S. patent application Serial Nos. 60/172,159, filed 17 Dec. 1999; 60/140,175, filed 18 June 1999; 60/172,154, filed 17 Dec. 1999; and 60/129,729, filed 16 Apr.1999, or to yield the corresponding motilides (see U.S. provisional patent application Serial No. 60/183,338, filed 18 Feb. 2000, attorney docket no. 30062-30053.00, inventors G. Ashley *et al.,*

In 43 of the 46 transformants that produced polyketides in the illustrative library described herein, the isolated polyketides had structures expected of the mutation(s). However, as observed in corresponding single mutations of the *eryA* gene in the single-plasmid system, additional products were observed with certain mutations. In most cases, the rapAT2 domain in module 3 recognized and processed both malonyl- and methylmalonyl-CoA and gave the expected 8-nor analogs plus lesser amounts of the 8-methyl analogs. In a few cases, only the 8-methyl analogs were formed. The relaxed-specificity of rapAT2 appears to be module-dependent, because only the expected products were observed with the same rapAT2 sequence at modules 2 or 5 (see Ruan *et al.,* 1997, Acyltransferase domain substitutions in erythromycin polyketide synthase yield novel erythromycin derivatives, J. *Bacteriol.* 179: 6416-25,

Likewise, when the KR of module 5 was replaced by either the rapDH/KR4 or rapDH/ER/KR1 domains to give the expected 4,5-anhydro and 5-deoxy analogs, respectively, 5-keto analogs were formed in addition to the expected products. This possibly results from transfer of the beta-ketothioester intermediate from KR5 to ACP5 at a rate competitive with its reduction by the heterologous rapKR4 domain. Because aberrant products were not observed with rapDH/KR4 at modules 2 or 6, the non-specificity appears to be module-dependent.

Finally, when the KR of module 2 was replaced with the rapDH/ER/KR1 domain to provide the 11-deoxy analogs, the 10,11-dehydro analogs were often observed as minor but significant products. Here, the intermediate is processed by the heterologous rapKR and DH domains in module 2, but transfer of the 10,11-dehydro intermediate to KS3 must be competitive with the ER-catalyzed reduction. Interestingly, with rapDH/ER/KR1 in module 2 and rapAT2 in module 3, the 10,11-dehydro by-products were not detected. Either the levels produced were too low for detection using the methods employed, or the aberrant dehydro intermediate of module 2 was not processed by module 3 containing the rapAT2 substitution.

Of the 18 transformants that did not produce polyketides at levels detectable in these experiments, two were double mutants and 16 were triple mutants; only one of these mutants was previously prepared in the single-plasmid system where it also failed to produce a detectable polyketide. As previously reported with the single-plasmid system, an increased number of mutations resulted in a decrease in yield to a level undetectable by the analytical method used.

A major advantage of the multiple plasmid system is that once multiple plasmids encoding functional mutants of PKS subunits are available, they can be rapidly combined with one or more additional mutants to expand the library of polyketides. In one example, a single Cys729Ala mutation at the KS1 domain of DEBS1 module 1 (see Jacobsen *et al.,1997, supra)* was prepared, yielding the KS1 null mutation. The inactive KS1 prevents propagation of the starter unit and permits introduction of exogenous synthetic diketide thiol esters into positions 12 and 13 of the 14-membered macrolide product. This system was first developed using a single vector, plasmid pJRJ2. Plasmid pJRJ2 encodes the *eryAI, eryAII,* and *eryAIII* genes; the *eryAI* gene contained in the plasmid contains the KS1 null mutation. The KS1 null mutation prevents formation of the 6-deoxyerythronolide B produced by the wild-type gene unless exogenous substrate is provided. Plasmid pJRJ2 and a process for using the plasmid to prepare novel 13-substituted erythromycins are described in PCT publication Nos. 99/03986 and 97/02358 and in U.S. patent application Serial Nos. 08/675,817, filed 5 July 1996; 08/896,323, filed 17 July 1997; and 09/311,756, filed 14 May 1999, The exogenous substrates provided can be prepared by the methods and include the compounds described in PCT patent application No. PCT/US00/02397 (Attorney Docket No. 30062-20032.40) and U.S. patent application Serial No. 09/492,733 (Attorney Docket No. 30062-20032.00), both filed 27 Jan. 2000, by inventors G. Ashley et al., and both of which claim priority to U.S. patent application Serial No. 60/117,384, filed 27 Jan. 1999,

The plasmid encoding the KS1 null allele of *eryAl* (Table 1) was introduced by co-transformation into *Streptomyces lividans* with the one *eryAII* mutant and seven *eryAIII* mutants (Table 1) to provide 16 transformants. Treatment of each of these with a propyl-diketide N-acetylcysteine thioester, following the work of Jacobsen *et al.,* 1997, and the methodology of the patent applications, *supra,* provided eleven novel 13-propyl polyketide analogs (49-59, Fig. 3). Thus, the present invention provides these novel polyketides as well as the compounds resulting from their modification by post-PKS tailoring enzymes (oxidases and glycosylases) or by synthetic chemical methods. To prepare these same PKSs by the single-plasmid system would have required preparation of 16 individual mutants rather than the single KS1 null mutant used in the present method.

In another example, the variants of *eryAI* and *eryAII* were used to prepare a small library of 12-membered macrolactones. It was previously shown in the *eryA* single-plasmid system that omission of module 6, along with fusion of module 5 to the thioesterase domain of DEBS3 (Fig.1A) results in formation of a 12-membered macrolactone. A truncated *eryAIII* gene containing module 5-TE (see *Kao et al.,* 1995, Manipulation of macrolide ring size by directed mutagenesis of a modular polyketide synthase, J. *Am. Chem. Soc.* 117: 9105-9106, and Table 1) was introduced into *Streptomyces lividans,* and the strain transformed with permutations of the four *eryAI* and two *eryAII* variants described above. Of the eight 12-membered lactones that could have been produced, five were observed **(44-48).** Compound **44** results from combining the wild-type *eryAI* and *eryAII* genes with the module 5-TE construct, and has previously been prepared in the single-plasmid system; compounds **45-48** are novel products that more than double the number of known 12-membered macrolides. These novel macrolactones constitute an important aspect of the present invention.

Other uses of this multiple plasmid system are as follows. The expression and genetic engineering of very large PKS genes, such as those involved in the biosynthesis of rapamycin (14 modules) or rifamycin (10 modules), is readily achievable by this method. The ORFs for each of the subunits of these and other PKSs, such as the mixed NRPS-PKS for epothilone (see U.S. patent application Serial No. 09/443,501, filed 19 Nov. 1999, and PCT patent application US99/27438, filed 19 Nov.1999, could be cloned and expressed in the manner used here to greatly simplify genetic manipulations and the structure/function analysis. Thus, in this application of the present method, a naturally occurring polyketide can be expressed in a recombinant host cell.

Similarly, it would be desirable to mix PKS genes from entirely different pathways to facilitate production of heterologous and hybrid PKSs, as precedented by the work of Li *et al.* involving hybrid erythromycin/ picromycin and oleandomycin/picromycinPKS genes (see U.S. patent application Serial Nos. 09/320,878, filed 27 May 1999, and 09/428,517, filed 28 Oct. 1999; PCT patent publication No. 99/61599; and PCT patent application No. US99/24478, each of

Such work could include extending the carbon chain by the addition of modules.

The PKS libraries generated could be leveraged and expanded by introducing genes for tailoring enzymes that oxidize, hydroxylate, methylate, acylate, glycosylate, or otherwise modify the product of the PKS or a modified polyketide, and genes encoding such enzymes could be employed using an additional vector in the multiple vector system. Various tailoring enzymes from different host organisms could be employed in the same system. Genes for such tailoring enzymes can be obtained as described in the patent applications and publications referenced in the preceding paragraph and elsewhere herein. See also, U.S. Patent No. 5,998,194,

Finally, the method should be useful with any system consisting of multimodular proteins, such as the large family of non-ribosomal peptide synthases, which produce many pharmaceutically important compounds such as anti-fungal compounds, anti-cancer compounds, and antibiotics. These important compounds are made by multifunctional synthetases, consisting of complexes of proteins containing between one and eleven modules, comparable to the modular PKSs (see *Konz et al.,* 1999, How do peptide synthase generate structural diversity? *Chem. & Biol.* 6: 39-48,

The multi-plasmid technology enables the realization of the full potential of modular PKSs and NRPSs, and thus libraries containing a complete repertoire of polyketides and non-ribosomal peptides. The achievement of this objective requires only the construction of a limited number of highly expressing, productive single mutants that will assure adequate polyketide production when the mutations are combined. Because all the elements for producing an extraordinarily large polyketide library are contained within this facile system, there is neither need nor benefit to embark on developing more complicated, less reliable systems to reach the same objective.

### Example 1

### Construction of Expression Plasmids

In each vector, the *eryA* gene is expressed under the control of the upstream *actI* promoter and *actII-ORF4* gene as previously described (see U.S. Patent No. 5,672,491 and Ziermann *et al.,* 2000, supra, both of which are ). For testing the high copy relicating plasmid possessing the pJV1 origin, the pB45 based *E*. *coli* shuttle vector pKOS025-32 was constructed by fusing pB45 with litmus 28 (New England Biolabs) at *Bgl*II and *PstI* sites. Into pKOS025-32 the ca. 14-kb *HindIII-XbaI* fragment containing the *actI* promoter and *actII*-ORF4 gene was inserted followed by the *eryAII* gene to give pKOS025-35. The configuration of the components in the *Hind*III-*Xba*I fragment is shown in Fig. 2. The same *Hind*III-*Xba*I fragment was also cloned into shuttle vector pOSint1/Hygro (see Raynal *et al.,* 1998, Structure of the chromosomal insertion site for pSAM2: functional analysis in *Escherichia coli, Molecular Microbiology* 28: 333-342, to yield the eryAIl/pSAM2-hyg plasmid, pKOS038-67..

### Example 2

### Transfer of Mutation Cassettes into the Three-plasmid System

The plasmids containing *eryAI* mutations in module 2 were pKOS025-179 (AT2→rapAT2), pKOS038-1 (KR→rapDH/KR4) and pKOS038-3 (KR→rapDH/ER/KR1) and were made as follows. The *Pac*I*-Spe*I fragments containing the corresponding mutations were transferred into the plasmids described in Fig. 2 from plasmids pKOS008-41, pKOS015-56 and pKOS015-57, respectively (see McDaniel et al., 1999, *supra,* and U.S. patent application Serial No. 09/429,349, filed 28 Oct. 1999, and PCT patent application US99/24483, filed 20 Oct. 1999, The plasmids containing the *eryAlll* mutations in module 5 were pKOS025-1831 (AT→rapAT2), pKOS025-1832 (KR→AT/ACP linker), pKOS025-1833 (KR→ rapDH/KR4) and pKOS025-1834 (KR→rapDHjERjKR1); and in module 6 were pKOS025-1841(KR- rapDH/KR4), pKOS021-106 (KR→AT/ACP linker) and pKOS025-1842 (AT→rapAT2). These were constructed as follows. The *Bgl*II*-Eco*RI fragments containing the corresponding mutations were transferred into the plasmids described in Fig. 2 from plasmids pKOS006-188, pKOS016-12, pKOS006-178, pKOS026-11b, pKOS011-25, pKOS011-13 and pKOS015-53, respectively (see McDaniel et al., 1999, *supra,* and U.S. patent application Serial No. 09/429,349, filed 28 Oct. 1999, and PCT patent application US99/24483, filed 20 Oct. 1999, ). Plasmid pKOS038-20 contains *eryAII* with the AT3→ rapAT2 replacement in module 3 and was made by transferring the mutation from a previously prepared sub-clone pKOS015-28 (see McDaniel et al., 1999, *supra,* and U.S. patent application Serial No. 09/429,349, filed 28 Oct. 1999, and PCT patent application US99/24483, filed 20 Oct. 1999,

### Example 3

### Streptomyces Transformation

*Streptomyces lividans* K4-114 and K4-155 (see U.S. patent application Serial No. 09/181,833, filed 28 Oct. 1998, and Ziermann *et al.,* 1999, Recombinant polyketide synthesis in *Streptomyces:* engineering of improved host strain, *BioTechniques* 26:106-110, transformants were prepared according to standard methods (see *Hopwood et al.,* 1985, *supra)* using apramycin (100 µg/mL), thiostrepton (50 µg/mL), and hygromycin (225 µg/mL) in the R5 protoplast regeneration plates. In the three-plasmid system for which eryAll/pSAM2-hyg replaced *eryAII*/SCP2*-hyg, *S*. *lividans* was transformed sequentially with pKOS010-153, pKOS038-67, and pKOS021-30.

### Example 4

### Diketide Feeding

The (2*S*, 3*R*)-2-methyl-3-hydroxyhexanoyl-N-acetylcysteamine (NAC) thioester (propyl diketide) was synthesized in accordance with the method described in U.S. patent application Serial Nos. 60/117,384, filed 27 Jan.1999, and Serial No. 09/492,733 (attorney docket no. 30062-20032.00), filed 27 Jan. 00, by the same inventors and claiming priority to the foregoing application. The *Streptomyces lividans* triple transformants containing the KS1 null allele of *eryAI* (see Jacobsen *et al.,* 1997, *supra,* and PCT patent publication Nos. 99/ 03986 and 97/02358, were cultured in 5 mL of R5 medium at 30°C for 6 days under appropriate antibiotic selection. On day 4 of the incubation, 300 µL of diketide solution (4.7 mg/mL in 10% DMSO) and 50 µL of pentanoic acid (2.5 mg/mL) were added to the culture.

### Example 5

### Production and Analysis of Polyketide Analogs

The *Streptomyces lividans* triple transformants were cultured in 5 mL of R5 medium (see Hopwood *et al.,* 1985, *supra)* at 30°C for 6 days under appropriate antibiotic selection. The cultured solution was extracted with 2 x 5 mL of ethyl acetate and the organic layers were combined and concentrated. A 50 µL aliquot of the concentrates was analyzed by HPLC on a reverse phase C₁₈ column (4.6 mm x 15 cm, Beckman, Fullerton, CA) using a PE SCIEX API100 LC/MS based detector (Perkin-Elmer, Foster City, CA). Quantitative determination of polyketide yield was made with evaporative light scattering detection (Analtec model 500 ELSD, Deerfield, IL). The polyketides were identified by their mass spectrum and correspondence to the products expected or to known standards. Under the ionization conditions used, 6dEB and its analogs generate signature dehydration patterns. The yield of the 13-ethyl 6dEB analogs varied from 7 mg/L to less than 0.1 mg/L and the 13-propyl analogs were produced in a range of 0.2 to 20 mg/L. The yields of the 12-membered lactones were not determined.

## Claims

1. A method for expressing a polyketide or non-ribosomal peptide in a host cell employing a multiplicity of recombinant vectors, which may be integrative or freely replicating, wherein each of the multiplicity of vectors encodes a portion of the polyketide synthase or non-ribosomal peptide synthase that produces the polyketide or non-ribosomal peptide, said method comprising the steps of introducing said vectors into said cell, and culturing said cell into which said vectors have been introduced under conditions such that said polyketide synthase or non-ribosomal peptide synthase is produced.

2. The method of claim 1, wherein said vectors encode a polyketide synthase.

3. The method of claim 2, wherein said polyketide synthase is selected from the group consisting of DEBS, an oleandolide PKS, a picromycin PKS, an epothilone PKS, a megalomicin PKS.

4. The method of claim 1, wherein said vectors encode a non-ribosomal peptide synthase.

5. The method of claim 1, wherein at least one of the multiplicity of vectors encodes a protein that further modifies the polypeptide or non-ribosomal peptide produced by said polyketide synthase or non-ribosomal peptide synthase.

6. The method of claim 1, wherein at least one of said vectors replicates extrachromosomally in said host cell.

7. The method of claim 1, wherein at least one of said vectors integrates into the chromosome of said host cell.

8. The method of claim 1, wherein said host cell is a *Streptomyces* host cell.

9. The method of claim 8, wherein said vector is a derivative of a vector selected from the group consisting of integrating vectors pSET152 and pSAM2 and extrachromosomally replicating vectors SCP2* and pJV1.

10. The method of any one of claims 1 to 9 further comprising the step of hydroxylation and/or glycosylation of the polyketide or non-ribosomal peptide.

11. A compound obtainable by the method of any one of claims 1 to 10 and selected from the group consisting of compounds shown in Figure 3 as compound nos. 29-33, 35-42 and 45-59.

12. The compound of claim 11 that is hydroxylated and/or glycosylated and is a 14-membered macrolactone with a C-6 hydroxyl and/or C-12 hydroxyl and/or a C-3 and/or C-5 glycosyl.

13. The compound of claim 12, wherein said glycosyl is either a desosaminyl residue at C-5 or a cladinosyl residue at C-3 or both.

14. The compound of claim 11 that is compound number 29 or 30 of Figure 3.

15. The compound of claim 11 that is compound number 31 or 32 of Figure 3.

16. The compound of claim 11 that is compound number 33 of Figure 3_

17. The compound of claim 11 that is compound number 35 or 36 of Figure 3.

18. The compound of claim 11 mat u compound number 37 or 38 of Figure 3.

19. The compound of claim 11 that is compound number 39 or 40 of Figure 3.

20. The compound of claim 11 that is compound number 41 or 42 of Figure 3.

21. The method of claim 1, wherein said each vector of said multiplicity of recombinant vectors is selected from a library of recombinant vectors comprising (i) a recombinant vector encoding a first functional mutant ORF and (ii) a recombinant vector encoding a second functional mutant ORF or a wild-type ORF.

## Patentansprüche

1. Verfahren zum Exprimieren eines Polyketids oder nicht ribosomalen Peptids in einer Wirtszelle unter Verwendung einer Vielzahl rekombinanter Vektoren, die integrativ oder frei replizierend sein können, wobei jeder aus der Vielzahl von Vektoren einen Abschnitt der Polyketidsynthase oder nicht ribosomalen Peptidsynthase kodiert, der das Polyketid oder nicht ribosomale Peptid produziert, wobei das genannte Verfahren die folgenden Schritte umfasst: Einleiten der genannten Vektoren in die genannte Zelle und Kultivieren der genannten Zelle, in die die genannten Vektoren eingeleitet wurden, unter solchen Bedingungen, dass die genannte Polyketidsynthase oder nicht ribosomale Peptidsynthase produziert wird.

2. Verfahren nach Anspruch 1, wobei die genannten Vektoren eine Polyketidsynthase kodieren.

3. Verfahren nach Anspruch 2, wobei die genannte Polyketidsynthase ausgewählt ist aus der Gruppe bestehend aus DEBS, einer Oleandolid-PKS, einer Picromycin-PKS, einer Fpvthilon-PKS, einer Megalomicin-PKS.

4. Verfahren nach Anspruch 1, wobei die genannten Vektoren eine nicht ribosomale Peptidsynthase kodieren.

5. Verfahren nach Anspruch 1, wobei wenigstens einer aus der Vielzahl von Vektoren ein Protein kodiert, das das Polypeptid oder nicht ribosomale Peptid weiter modifiziert, das von der genannten Polyketidsynthase oder nicht ribosomalen Peptidsynthase produziert wird.

6. Verfahren nach Anspruch 1, wobei sich wenigstens einer der genannten Vektoren in der genannten Wirtszelle extrachromosomal repliziert.

7. Verfahren nach Anspruch 1, wobei sich wenigstens einer der genannten Vektoren in das Chromosom der genannten Wirtszelle integriert.

8. Verfahren nach Anspruch 1, wobei die genannte Wirtszelle eine *Streptomyces* Wirtszelle ist.

9. Verfahren nach Anspruch 8, wobei der genannte Vektor ein Derivat eines Vektors ist, der ausgewählt ist aus der Gruppe bestehend aus den sich integrierenden Vektoren pSET152 und pSAM2 und sich extrachromosomal replizierenden Vektoren SCP2* und pJV1.

10. Verfahren nach einem der Ansprüche 1 bis 9, das ferner den Schritt des Hydroxylierens und/oder Glykosylierens des Polyketids oder nicht ribosomalen Peptids umfasst.

11. Verbindung, die durch das Verfahren nach einem der Anspruche 1 bis 10 erhältlich und ausgewählt ist aus der Gruppe bestehend aus den in Fig. 3 dargestellten Verbindungen Nr. 29-33, 35-42 und 45-59.

12. Verbindung nach Anspruch 11, die hydroxyliert und/oder glykosyliert ist und ein 14-gliedriges Makrolacton mit einem C-6 Hydroxyl und/oder C-12 Hydroxyl und/oder einem C-3 und/oder C-5 Glycosyl ist.

13. Verbindung nach Anspruch 12, wobei das genannte Glycosyl entweder ein Desosaminylrest an C-5 oder ein Gladinosyl-Rest an C-3 oder beides ist.

14. Verbindung nach Anspruch 11, die die Verbindung Nummer 29 oder 30 aus Fig. 3 ist.

15. Verbindung nach Anspruch 11, die die Verbindung Nummer 31 oder 32 aus Fig. 3 ist.

16. Verbindung nach Anspruch 11, die die Verbindung Nummer 33 aus Fig. 3 ist.

17. Verbindung nach Anspruch 11, die die Verbindung Nummer 35 oder 36 aus Fig. 3 ist.

18. Verbindung nach Anspruch 11, die die Verbindung Nummer 37 oder 38 aus Fig. 3 ist.

19. Verbindung nach Anspruch 11, die die Verbindung Nummer 39 oder 40 aus Fig. 3 ist.

20. Verbindung nach Anspruch 11, die die Verbindung Nummer 41 oder 42 aus Fig. 3 ist.

21. Verfahren nach Anspruch 1, wobei jeder genannte Vektor aus der genannten Vielzahl von rekombinanten Vektoren ausgewählt ist aus einer Bibliothek rekombinanter Vektoren, der (i) einen rekombinanten Vektor, der ein erstes funktionelles Mutanten-ORF kodiert, und (ii) einen rekombinanten Vektor umfasst, der ein zweites funktionelles Mutanten-ORF oder ein Wildtyp-ORF kodiert.

## Revendications

1. Procédé pour exprimer un polykétide ou un peptide non ribosomique dans une cellule hôte employant une multiplicité de vecteurs recombinants, qui peuvent être intégratifs ou se répliquer librement, dans lequel chacun parmi la multiplicité de vecteurs code une partie de la polykétide synthase ou de la peptide non ribosomique synthase qui produit le polykétide ou le peptide non ribosomique, ledit procédé comprenant les étapes consistant à introduire lesdits vecteurs dans ladite cellule, et à cultiver ladite cellule dans laquelle lesdits vecteurs ont été introduits dans des conditions telles que ladite polykétide synthase ou ladite peptide non ribosomique synthase est produite.

2. Procédé selon la revendication 1, dans lequel lesdits vecteurs codent une polykétide synthase.

3. Procédé selon la revendication 2, dans lequel ladite polykétide synthase est choisie parmi le groupe constitué de la DEBS, d'une oléandolide PKS, d'une picromycine PKS, d'une épothilone PKS, d'une mégalomicine PKS.

4. Procédé selon la revendication 1, dans lequel lesdits vecteurs codent une peptide non ribosomique synthase.

5. Procédé selon la revendication 1, dans lequel au moins un parmi la multiplicité de vecteurs code une protéine qui modifie en outre le polypeptide ou le peptide non ribosomique produit par ladite polykétide synthase ou ladite peptide non ribosomique synthase.

6. Procédé selon la revendication 1, dans lequel au moins un desdits vecteurs se réplique de façon extrachromosomique dans ladite cellule hôte.

7. Procédé selon la revendication 1, dans lequel au moins un desdits vecteurs s'intègre dans le chromosome de ladite cellule hôte.

8. Procédé selon la revendication 1, dans lequel ladite cellule hôte est une cellule hôte de *streptomycète.*

9. Procédé selon la revendication 8, dans lequel ledit vecteur est un dérivé d'un vecteur choisi parmi le groupe constitué des vecteurs d'intégration pSET152 et pSAM2 et des vecteurs de réplication extrachromosomique SCP2* et pJV1.

10. Procédé selon l'une quelconque des revendications 1 à 9, comprenant en outre l'étape d'hydroxylation et/ou de glycosylation du polykétide ou du peptide non ribosomique.

11. Composé pouvant être obtenu par le procédé selon l'une quelconque des revendications 1 à 10 et choisi parmi le groupe constitué des composés présentés dans la figure 3 tels que les composés n° 29 à 33, 35 à 42 et 45 à 59.

12. Composé selon la revendication 11, lequel est hydroxylé et/ou glycosylé et qui est une macrolactone à 14 chaînons avec un groupe hydroxy en position C₆ et/ou un groupe hydroxy en position C₁₂ et/ou un groupe glycosyle en position C₃ et/ou un groupe glycosyle en position C₅.

13. Composé selon la revendication 12, dans lequel ledit groupe glycosyle est soit un résidu de désosaminyle situé en position C₅, soit un résidu de cladinosyle situé en position C₃, soit les deux.

14. Composé selon la revendication 11, lequel est le composé numéro 29 ou 30 de la figure 3.

15. Composé selon la revendication 11, lequel est le composé numéro 31 ou 32 de la figure 3.

16. Composé selon la revendication 11, lequel est le composé numéro 33 de la figure 3.

17. Composé selon la revendication 11, lequel est le composé numéro 35 ou 36 de la figure 3.

18. Composé selon la revendication 11, lequel est le composé numéro 37 ou 38 de la figure 3.

19. Composé selon la revendication 11, lequel est le composé numéro 39 ou 40 de la figure 3.

20. Composé selon la revendication 11, lequel est le composé numéro 41 ou 42 de la figure 3.

21. Procédé selon la revendication 1, dans lequel chaque vecteur parmi la multiplicité de vecteurs recombinants est choisi à partir d'une banque de vecteurs recombinants comprenant (i) un vecteur recombinant codant un premier ORF mutant fonctionnel et (ii) un vecteur recombinant codant un second ORF mutant fonctionnel ou un ORF de type sauvage.
